(19) Europäisches Patentamt
European Patent Office
Office européen des brevets

(11) **EP 3 199 231 B1**

(12) **EUROPEAN PATENT SPECIFICATION**

(45) Date of publication and mention
of the grant of the patent:
**17.02.2021 Bulletin 2021/07**

(51) Int Cl.:
**B01J 8/02** (2006.01)    **C07C 29/151** (2006.01)
**C07C 31/04** (2006.01)   **C07D 301/10** (2006.01)

(21) Application number: **15845340.7**

(22) Date of filing: **23.09.2015**

(86) International application number:
**PCT/CN2015/090332**

(87) International publication number:
**WO 2016/045585 (31.03.2016 Gazette 2016/13)**

(54) **LARGE REACTOR AND DEVICE AND PROCESS THEREOF**

GROSSER REAKTOR SOWIE VORRICHTUNG UND VERFAHREN DAFÜR

RÉACTEUR DE GRANDE TAILLE ET DISPOSITIF ET PROCÉDÉ CORRESPONDANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.09.2014 CN 201410497496**

(43) Date of publication of application:
**02.08.2017 Bulletin 2017/31**

(73) Proprietor: **Lou, Ren
Hangzhou
Zhejiang 310012 (CN)**

(72) Inventors:
• **LOU, Ren
Hangzhou
Zhejiang 310012 (CN)**
• **LOU, Shoulin
Hangzhou
Zhejiang 310012 (CN)**

(74) Representative: **V.O.
P.O. Box 87930
Carnegieplein 5
2508 DH Den Haag (NL)**

(56) References cited:
**EP-A1- 0 534 195      EP-A1- 0 567 273
EP-A2- 0 336 440      CN-A- 1 117 490
CN-A- 1 174 096       CN-A- 1 355 725
CN-A- 1 363 416       CN-A- 101 429 098
CN-A- 103 240 036     CN-A- 103 373 898
CN-A- 103 524 299     CN-U- 202 460 592
CN-U- 202 747 831     CN-U- 203 096 014
CN-U- 204 365 252     DE-A1-102012 220 926
GB-A- 1 321 095       US-A- 4 525 482
US-A- 5 233 060**

• **Shahin Shamsi ET AL: "Optimization of Steam
Pressure Levels in a Total Site Using a
Thermoeconomic Method", Energies, vol. 5, no.
3, 12 March 2012 (2012-03-12), pages 702-717,
XP055632873, DOI: 10.3390/en5030702**

**Description**

**Technical Field**

The disclosure is directed to the field of oil and coal chemical industry, particularly to fluid fixed-bed catalytic reactions and heat transfer processes, even particularly to strong exothermic and endothermic chemical reactions of methanol, ethylene oxide, ethylene glycol, methanation, dimethyl ether, hydrocarbons and the like in petrochemical industry and coal chemical industry.

**Background Art**

The coal chemical industry in China has attracted attention around the world nowadays for the following reasons. First, the development is rapid, and the investments are tremendous. Second, the scale is large. National Development and Reform Commission places requirements on the scale of new coal chemical projects. For example, the yield for synthesis of methanol should be 1 million tons per year; and the yield for synthesis of ethylene glycol should be 0.2 million tons per year. Some well-known foreign companies such as Lurgi, DAVY, among others, each have succeeded in development of processes for synthesis of megamethanol at 5000 tons per day, and these processes have been put into operation in the coal chemical devices of Shenhua Ningxia Coal Industry Group Corporation and Baotou, etc in China. Third, foreign technologies are utilized in all the large reactors in the coal chemical projects. Fourth, a combination of several reactors is employed in the large-scale coal chemical projects due to the low capability of a single reactor.

Up to date, multinational companies have developed a number of types of reactors successfully. For the most commonly used fixed-bed reactor, there are adiabatic type, quenching type, water or air cooling heat exchange type, tubular or plate heat exchange type, axial or radial type, vertical or horizontal type reactors. The DAVY radial converter has low resistance, but its capability of heat transfer is insufficient. As a result, a hot spot in operation has a temperature up to more than 300°C, affecting the service life of a catalyst. Both the Lurgi water cooling and air cooling converters in tandem for megamethanol are radial converters, wherein the pressure drop in the synthesis converter and the resistance in the synthesis loop are large. The total resistance is up to more than 0.7MPa, and thus the power consumption is increased.

WO2007021393 and CN101243027 to Exxon-mobil Chemical Corporation, USA disclose a large reaction system for synthesis of methanol, consisting of a tube-shell reactor, a gas cooling tube and a catalytic reactor external to a water cooling tube in series. The diameter is 5.8 m, and the total height of the bed is 22 m, and the single path conversion of CO is 35.3%. This system also has the problem that the larger the reactor is, the larger the resistance is in synthesis.

During upsizing of both domestic and foreign petrochemical engineering projects, a conflict between a high heat transfer efficiency and a high catalyst loading coefficient occurs in the design of an industrial catalytic reactor. For example, a tube-shell reactor has good capability of heat transfer, and has been used widely as a large reactor for synthesis of methanol and ethylene oxide in tens of years. However, as the expensive catalyst is loaded in a reaction tube of 2-5 cm, the catalyst space effectively used for the reaction is generally not higher than a third of the whole reactor. For example, in Yangzi Petrochemical Company Ltd, China, a tube-shell reactor for synthesis of ethylene oxide at a yield of 0.18 million tons per year has a diameter up to 7 meters, a length of 22 meters, more than 15000 reaction tubes, and a weight up to 850 tons. From design, manufacture, processing to shipment and installation, several units made great joint efforts to address the challenges, so that the project was finished smoothly. In addition, except for adiabatic type and quenching type reactors, the internal members of all water or gas cooling heat exchange type reactors include a large number of heat exchangers of the type of heat exchange tube that are welded to a main for inlet and outlet of water or gas, or a good number of catalyst loaded reaction tubes welded to a large tube sheet. When the reactors are put into operation, the welding spots likely crack due to variation of the operating conditions such as temperature, stress, etc, leading to leak which will affect the production.

All in all, no matter at home or abroad, there is so far no large methanol synthesis converter having good process performances, a uniform temperature, a high conversion, a high space utility, a high energy efficiency, and a yield of 1 million tons per year per unit.

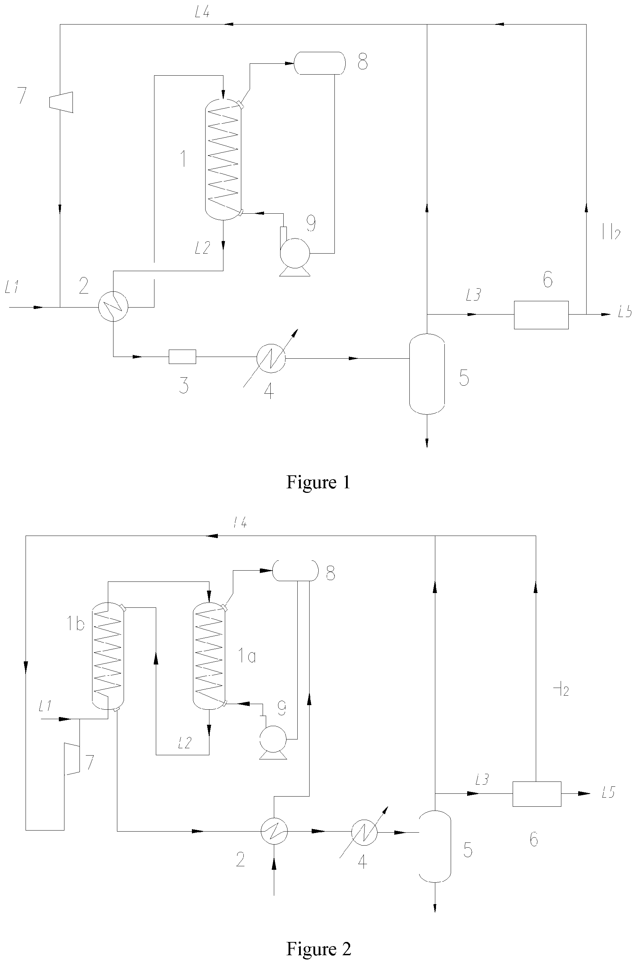

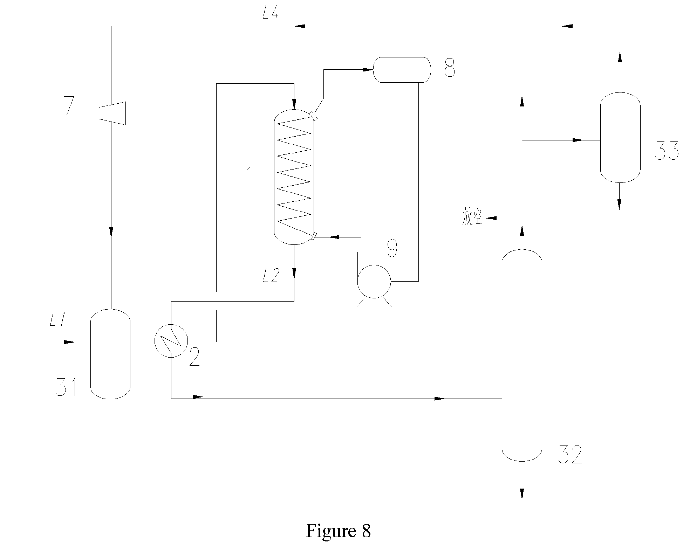

CN 202 460 592 U (hereinafter CN '592) and CN 103 240 036 (hereinafter CN '036) were applications for utility model and for invention, filed by the same Applicant on the same filing date. As described in Line 4 of claim 1 of CN '592 and CN '036, the bottoms of two adjacent inner and outer ring heat exchange tubes or two adjacent heat exchange tubes of the same ring diameter are connected by a U-shaped bend to form an upward heat exchange tube and a downward heat exchange tube arrangement for internal heat exchange medium. The upward heat exchange tube is connected to the outlet tube case, and the downward heat exchange tube is connected to the inlet tube case; or the tops of two adjacent inner and outer ring heat exchange tubes or two adjacent heat exchange tubes of the same ring diameter are connected by an inverted U-shaped bend, and the upward heat exchange tube connecting the inverted U-shaped bend is connected to the inlet tube case, and the downward heat exchange tube is connected to the outlet tube case". The structures of the U-shaped bend and the inverted U-shaped bend are described in detail in Figures 8-9 of CN '592 and CN '036. The water-cooled winding tube case is set on the upper or lower part of the shell. As shown in Figure 1 of CN '592 and CN '036, the inlet tube case is set on the upper part. The water within the tube flows through the tube 2b and flows downward with the reaction gas outside the tube, and then changes its direction via the U-shaped bend 18a, and then flows upward through the tube 2a, in a direction contrary to the reaction gas stream outside the tube (see Fig. 8 of CN '592 and CN '036). Or, as shown in Figure 2 of CN '592 and CN '036, the inlet tube case is located at the lower part, and connected to the lower inlet tube 2a. Water flows upward, in contrary direction with the reaction gas outside the tube, to the tube end, and then flows downwards via the inverted U-shaped bend 18b to the tube 2b (see Figure 9 of CN '592 and CN '036).

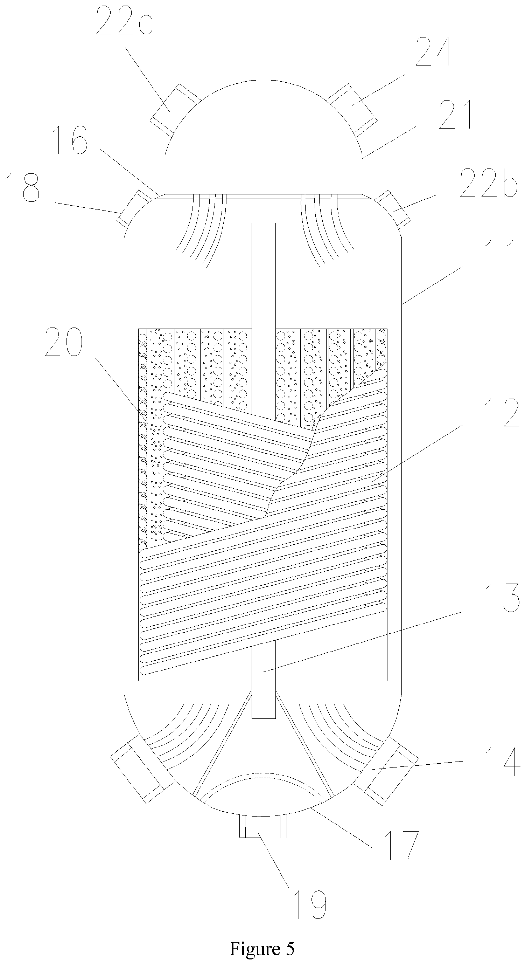

CN 203 096 014 U (hereinafter CN '014) relates to a device for producing natural gas using water gas. As shown in the description of Example 3 of CN '014 and Figure 5 of CN '014, it uses the same structure as CN '592 and CN '036.

In CN '592, CN '014 and CN '036, the inlet and outlet tube cases of the water-cooled winding tube heat exchanger reactor are all set at the upper end of the shell, or the inlet and outlet tube cases are all at the lower end of the shell. The water in the inlet tube changes its direction via the U-shaped or inverted U-shaped bend located in the middle of the tube.

## Summary

In view of the drawbacks of the reaction technologies and reactors for large-scale processes in the prior art, the disclosure proposes a solution for the reaction technologies and reactors for large-scale processes, featuring a uniform temperature, a high efficiency and a low pressure drop.

To achieve the above object, the present invention provides a large reactor according to the appended independent claim 1, and a large-scale process according to the appended independent claim 7. Preferable embodiments of the invention are provided by the appended dependent claims 2-6 and 8-9.

The disclosure utilizes the following technical solution:

A large reactor, comprising a housing, an internal heat-exchange member, an inlet tube case and an outlet tube case, wherein the internal heat-exchange member comprises a heat-exchange winding tube; wherein the housing comprises an upper seal head, a cylinder body, a lower seal head; wherein a gas inlet and a gas outlet are provided on the upper seal head and the lower seal head respectively; wherein the numbers of the inlet tube case and/or the outlet tube case each are 2 or a multiple of 2, which are respectively provided on the lower seal head and the upper seal head, or on a lower part and an upper part of the cylinder body, arranged uniformly in a circumferentially symmetric manner, which facilitates uniform distribution and flow of a reaction gas in a catalyst layer in a heat-exchange tube in the reactor, improves reaction efficiency, and effects easy discharge of the catalyst from a bottom; wherein a small diameter tube case tube sheet is used to replace a large tube case tube sheet to reduce a thickness of the tube sheet, so as to save a device material and cost. The inlet tube case and the outlet tube case are connected to two ends of the heat-exchange winding tube respectively, wherein the heat-exchange winding tube is separated and positioned by a positioning strip and winds into a layer-by-layer tube set, wherein a catalyst is loaded between heat-exchange winding tubes to form a catalyst layer.

A steam drum is provided on the upper seal head, also used as an outlet tube case, wherein an even number of winding tube sets in a sectorial tube distribution zone are connected to a tube sheet between the steam drum and the upper seal head.

Preferably, the large reactor is a radial-axial reactor, wherein a porous gas collecting cylinder is provided at a middle-to-lower part of an inner wall of the cylinder body, and a central gas distributing cylinder is provided at a center of the cylinder body, wherein the central gas distributing cylinder has pores at a middle-to-lower part, wherein a stainless steel screen is laid on the central gas distributing cylinder and the porous gas collecting cylinder at a side adjacent to the catalyst, wherein the catalyst layer external to the heat-exchange winding tube is between the central gas distributing cylinder and the porous gas collecting cylinder, wherein the gas outlet is positioned on the upper seal head, wherein the gas inlet is positioned at a top of the central gas distributing cylinder, wherein the central gas distributing cylinder is a hollow mandrel with its bottom sealed. The reactor in this configuration is a reactor with tubes wound radially first and then axially, wherein a gas enters from the gas inlet at a top of the central gas distributing cylinder and is distributed uniformly through the distributing pores at the middle-to-lower part of the central gas distributing cylinder into the catalyst layer, wherein most of the gas passes radially through the catalyst layer, enters the porous gas collecting cylinder, and flows upward axially in the porous gas collecting cylinder, wherein the reaction gas exiting the porous gas collecting cylinder continues flowing upward axially to leave the reactor from the gas outlet.

Preferably, the large reactor is an axial-radial reactor, wherein a porous gas distributing cylinder is provided at a middle-to-lower part of an inner wall of the cylinder body, wherein the middle-to-lower part accounts for 90-95% of a length of the inner wall starting from a lower end, wherein a central gas collecting cylinder is provided at a center of the cylinder body, wherein the central gas collecting cylinder has pores at a middle-to-lower part which accounts for 90-95% of a length of the central gas collecting cylinder starting from a lower end, wherein a stainless steel screen is laid on the central gas collecting cylinder and the porous gas distributing cylinder at a side adjacent to the catalyst layer, wherein the central gas collecting cylinder is fixed at a center of the cylinder body with a positioning ring, wherein the catalyst layer external to the heat-exchange winding tube is between the central gas collecting cylinder and the porous gas distributing cylinder, wherein the gas inlet is positioned on the upper seal head, wherein the gas outlet is positioned at a top of the central gas collecting cylinder. The reactor in this configuration is a reactor with tubes wound axially first and then radially, wherein a gas enters from the gas inlet on the upper seal head, wherein most of the gas flows downward along a gas distributing passage between the housing and the porous gas distributing cylinder, and is distributed uniformly through the distributing pores in the porous gas distributing cylinder into the catalyst layer, flows radially through the catalyst layer, and enters the central gas collecting cylinder from collecting pores in the central gas collecting cylinder, wherein a small portion of the gas flows directly downward axially in an annular space extending along 10% of a height of a bed layer from a top thereof between the porous gas distributing cylinder 27 and the central gas collecting cylinder 28, and flows inward radially to converge in the inner gas collecting cylinder, and then flows upward in the central gas collecting cylinder to exit the reactor from the gas outlet at the top.

Preferably, the heat-exchange winding tube is a small-size spiral heat-exchange tube having an inner diameter of 10-25 mm.

Preferably, a spacing of two axially adjacent tube walls of a portion of the heat-exchange winding tube extending along 10-50% of a height of the catalyst bed layer from a top thereof is 10-90% of a normal uniform spacing.

The reaction rate of a gas in the catalyst bed layer and the released reaction heat vary, i.e. the reaction rate is higher in the front part of the reactor than at the back part of the reactor, and the released reaction heat is more in the front part of the reactor than at the back part of the reactor. When a tube-shell reactor is used, the heat-exchange area is the same, and thus a peak reaction temperature occurs in the front part of the bed layer. For this reason, the spacing of two vertically adjacent tube walls of the heat-exchange tube is reduced in the front part of the catalytic reactor according to the disclosure. For example, the tube wall spacing of a 10-50% portion of the bed layer starting from the gas inlet is reduced by 10-90% to enlarge the heat-exchange area and increase the heat removal rate. As such, the problems of overheating of hot spots in the catalyst layer and deactivation caused by unduly high temperatures are solved, and the service life of the catalyst is extended.

Preferably, a spacing of two axially adjacent tube walls of a portion of the heat-exchange winding tube extending along 20% of a height of the catalyst bed layer from a bottom thereof is 10-90% of a normal uniform spacing. At the bottom of the catalytic reaction bed layer, the product content in the reaction gas is high, and the temperature of the reaction gas is close to the equilibrium temperature of the product for an exothermic reaction. In order to further increase the product content, the tube wall spacing can also be reduced. For example, the tube wall spacing of a portion of the heat-exchange tube located deeper than 80% of the bed layer can be reduced by 10-90%, so as to remove more heat and reduce the reaction temperature, which is helpful for further increasing the product content.

Preferably, the heat-exchange winding tube set is separated by an intermediate sleeve or a perforated sleeve to provide an inner catalyst layer and an outer catalyst layer, wherein an end of the intermediate sleeve or perforated sleeve is connected to the seal head via a plurality of supporting rods, wherein the intermediate sleeve or perforated sleeve has a diameter of 1/2 to 1/3 of the diameter of the housing.

Preferably, the large reaction device comprises a single large axial water cooling tube wound reactor, or two or three large axial water cooling tube wound reactors in parallel connected to a large gas-cooling reactor in series.

The large reactor described above can be used in exothermic or endothermic reactions such as conversion of carbon monoxide from synthetic gas to carbon dioxide and hydrogen, oxidation of hydrogen sulfide to prepare sulfur, synthesis of oil by Fischer-Tropsch reaction, methanation, production of ethylene oxide and ethylene glycol, synthesis of ammonia, synthesis of methylamine from methanol and ammonia, etc.

The large multi-stage methanol reaction device consists of two large methanol synthesis units in series, wherein each large methanol synthesis unit comprises a large water-cooling reactor, a heat exchanger, an air or water cooler, a methanol separator and a circulator, wherein a fresh raw gas is all fed to a first methanol synthesis unit, wherein, except for a portion of an export gas from the methanol separator that is recycled through the circulator at a circulating ratio <0.5 to the large reactor for further synthesis of methanol, the rest released gas is discharged into the second methanol synthesis unit at the back in series at a circulating ratio of 1.5-4 for synthesis of methanol, wherein a released gas from the second stage synthesis unit is delivered to an effective gas (hydrogen) recycling device to recycle hydrogen, wherein the reclaimed hydrogen is merged with the fresh raw gas, and then pressurized and delivered to the first synthesis unit for synthesis of methanol.

A large-scale process for synthesis of methanol using the above large reactor at a low circulating ratio of <3 and a high net value of >10%, including the following steps:

1) mixing a fresh raw gas with hydrogen reclaimed from a released gas from the synthesis to form a mixed gas,

$$\frac{(H_2 - CO_2)}{(CO + CO_2)} = 2.0\text{-}2.05,$$

wherein wherein the mixed gas is purified deeply, mixed with a circulating gas, and then enters a large reactor for synthesis of methanol;

2) raising a temperature of the mixed gas to above 210°C by heat exchange in a heat exchanger, wherein reaction heat from the synthesis of methanol heats up water in a heat-exchange winding tube to produce a medium-pressure steam, wherein methanol in a reaction gas arriving at an outlet has a molar percentage of >10%;

3) cooling a hot gas having a high content of methanol exiting the large reactor by heat exchange in a heat exchanger, wherein cooling is achieved by heating water in a boiler or with an air cooler, wherein the gas is then cooled to about 40 °C by water cooling, and then delivered to an alcohol separator for isolation of methanol;

4) recycling the reaction gas after product separation through a circulator to undergo further reaction, wherein the ratio of the circulating gas to the fresh raw gas, namely the circulating ratio, is less than 3, wherein, as another part, the released gas is subjected to membrane separation, pressure swing absorption or the like to recycle effective hydrogen which returns to synthesize methanol, or enters a next methanol synthesis reaction loop for further reaction to form methanol, or the released gas is used for co-production of other products or for power generation.

A large-scale process for synthesis of ethylene oxide by ethylene oxidation using the above large reactor, including the following steps:

1) adding a $CH_4$ stabilizer and pure oxygen, or adding air to an ethylene raw material purified to remove explosive alkane components including $H_2$, alkenes of $C_3$ or above to obtain a mixed raw gas comprising mainly ethylene 20-30%, oxygen 7-10%, methane 45-63%, or introducing nitrogen by oxidation with air;

2) mixing the raw gas and a circulating gas, wherein the resulting mixture is heated to about 180 °C with an in-and-out-of-tower heat exchanger at a pressure level of 2 MPa, and then enters a large reactor for synthesis of ethylene oxide;

3) allowing the gas entering the tower to undergo reaction of ethylene oxidation to produce ethylene oxide on a gaseous phase silver catalyst comprising 10-20wt% silver and the balance of a co-catalyst and a support between a heat-exchange tube and a housing of the reactor, wherein oxidation reaction heat is absorbed by water in the tube to produce a medium-pressure steam, wherein a temperature of the oxidation reaction is controlled by controlling a pressure of the steam byproduct;

4) lowering a temperature of a reaction gas exiting the reactor by cooling it as the reaction gas heats a gas entering the reactor with heat transferred in a hot gas passage in a heat exchanger;

5) allowing the reaction gas exiting the heat exchanger to enter an absorption tower to absorb ethylene oxide as a product, wherein a $C_2H_4O$ rich liquid at a bottom of the absorption tower is delivered to a post treatment station, wherein a small amount of released gas is discharged intermittently from a gas at a top of the absorption tower to avoid inert gas accumulation, and a small part of the gas passes through a $CO_2$ removing tower and is pressurized in a circulator together with the rest of the gas without removal of $CO_2$, wherein the pressurized gas returns to merge with the mixed raw gas in front of the reactor and then enters the reactor for synthesis of ethylene oxide.

The invention possesses outstanding advantages as compared with the prior art, and a specific comparison is shown in the table below:

Economical comparison between the main technical apparatuses for producing 5000 tons/day megamethanol

| Developer | British DAVY | German Lurgi | British JOHSON MATTHEY | American EXXON MOBIL | The invention | |
|---|---|---|---|---|---|---|
| Technical characteristics | Water cooling tube, radial tower in series/parallel | Two tube-shell in parallel Water cooling-gas cooling in series | Two adiabatic towers and one gas cooling tower in series | Water cooling tube shell; gas cooling tube tower, water cooling tube tower in series | Single water cooling winding tube | |
| | | | | | Example 7 | Example 8 |
| Output (ton/day) | 5400 | 5000 | 2700 | 6000 | 5000 | 10000 |

(continued)

| Developer | British DAVY | German Lurgi | British JOHSON MATTHEY | American EXXON MOBIL | The invention | |
|---|---|---|---|---|---|---|
| Technical characteristics | Water cooling tube, radial tower in series/parallel | Two tube-shell in parallel Water cooling-gas cooling in series | Two adiabatic towers and one gas cooling tower in series | Water cooling tube shell; gas cooling tube tower, water cooling tube tower in series | Single water cooling winding tube | |
| | | | | | Example 7 | Example 8 |
| Synthesis pressure (MPa) | 8.0 | 8.5 | 8.4 | 4.7 | 8.2 | 8.2 |
| Synthesis tower diameter * height | 5.2*10 | 4*10 | | one 5.5*8 tower two 5.8*7 towers | 5.8*7.5 | 5.4*18.8 |
| Number of synthesis towers (unit) | 2 | 3 | 3 | 3 | 1 | 2 |
| Empty capacity of the synthesis tower ($M^3$) | 390 | | 215 | 560 | | |
| Total amount of the catalyst ($M^3$) | 205 | 180 | 116.4 | about 335 | 172 | 356 |
| Heat exchanges (unit) | 2 | 1 | 3 | 1 | 1 | 1 |
| Water cooling alcohol separator (unit) | 2 | 1 | 1 | 1 | 1 | 1 |
| Circulator (unit) | 1 | 1 | 1 | 1 | 1 | 1 |
| Circulating ratio | 5.6 | 1.5 | 3 | | 1.3 | 1.47 |
| Alcohol net value (%) | <5 | 16.3 | 6.4 | | 19 | 17.2 |
| Pressure drop in the synthesis loop (MPa) | 0.9 | 0.76 | | | low $\leqq$ 0.6 | $\leqq$ 0.6 |
| Synthesis temperature (°C) | high >300°C | 209-280°C | large temperature difference | front tower 247-283°C back Tower 170-210°C | 220-270°C | 220-270°C |
| Catalyst life | relatively short | long | relatively short | medium | long | long |
| Byproduct of methanol | relatively high | low | medium | medium | low | low |

(continued)

| Developer | British DAVY | German Lurgi | British JOHSON MATTHEY | American EXXON MOBIL | The invention | |
|---|---|---|---|---|---|---|
| Technical characteristics | Water cooling tube, radial tower in series/parallel | Two tube-shell in parallel Water cooling-gas cooling in series | Two adiabatic towers and one gas cooling tower in series | Water cooling tube shell; gas cooling tube tower, water cooling tube tower in series | Single water cooling winding tube | |
| | | | | | Example 7 | Example 8 |
| Raw gas consumption Nm$^3$/ton | 2330 | 2338 | 3291 | | 2265 | 2265 |
| Byproduct steam /ton alcohol | low, <ton/T | low, 0.8 ton/T | producing hot water | <0.8 ton/T | $\geqq$ 1.3 | $\geqq$ 1.3 |
| Investment | 100 | 100 | 85 | 90 | 70 | 60 |
| Energy consumption | relatively high | medium | relatively high | medium | low | low |
| Source | Chemical Engineering Design Communication , p. 58, December, 2013 CN101301597A | balance sheet of materials for methanol production from Datang Duolun | WO200406 5341 | CN1012430 27 | | |
| Amount of catalyst (M$^3$) when normalized to 5000 tons/day | 197 | 180 | 215 | 194 | 172 | 178 |

As can be seen from above, the main advantages include:

1. Easy scaling up: as the temperature is homogeneous, the activity of the catalyst can be improved significantly. In addition, the catalyst loading coefficient is doubled as compared with the tube-shell type. Hence, scaling up can be realized easily. For example, one methanol tower can replace several synthesis towers of foreign companies Lurgi, DAVY and the like using megamethanol technologies.

2. Energy can be saved, and consumption can be reduced. The synthesis conversion is high, while the consumption of raw materials is low. The amount of the byproduct steam is high, and the recovery of heat is excellent. The circulating ratio is small, the power consumption is low, and the operating cost is also low.

3. Low investment: the tubes in the device are small; the footprint area is small. The investment is low, as one unit has the effect of two prior art units. The catalyst loading coefficient in the synthesis tower is high, and the catalyst per unit is highly active. The efficiency and the yield are high. The reaction heat can be used effectively. A large amount of steam is produced from the reactor. The recovery of the heat is high. The circulating ratio is low, and the net value of the product is high. The resistance is small, and the power and energy consumption is low.

4. The structure is safe and reliable. As the internal members are free of welding spots, and the elastic structure of the spiral heat-exchange tube has no temperature stress, the safety and reliability of the structure of the synthesis tower is guaranteed fundamentally, and there is no risk of leak. The welding spots of the tubes to the tube sheet are located at the tube case on the housing, and thus inspection and repairing have no influence on the catalyst activity. As such, the challenges of manufacture, assembly, maintenance and shipment of large towers are addressed successfully with high security and reliability.

5. Optimized design and easy scaling up: with advanced designing means mastered, a mathematical model has been constructed for a tube wound synthesis tower, which is suitable for various production capacities and process conditions and parameter calculations to optimize structural parameters, so as to achieve the best effects. As there are no large tube sheets or baffle plates, and a structure featuring small heat-exchange tube cases and positioning strips is used, the challenge of processing a large diameter tower is addressed.

## Description of the Drawings

Fig. 1 is a schematic view showing a large loop for synthesis of methanol, consisting of a single vertical synthesis tower with an axial water cooling winding tube, and devices for heat exchange, separation and circulation, etc.
Fig. 2 shows an assembly of a water cooling tube wound tower and a gas cooling tube wound tower in series.
Fig. 3 shows an axial water cooling tube wound reactor comprising an even number of paired inlet and outlet tube cases.
Fig. 4 shows an axial water cooling tube wound reactor with an intermediate sleeve provided.
Fig. 5 shows an axial water cooling tube wound reactor with a steam drum provided on an upper seal head on the synthesis tower.
Fig. 6 shows an axial-radial tube wound reactor with radial winding followed by axial winding.
Fig. 7 shows an axial-radial tube wound reactor with axial winding followed by radial winding.
Fig. 8 shows a process flow chart of a large-scale process for synthesis of ethylene oxide by oxidation of ethylene.

Notes on the reference numbers:
1- reactor 2-heat exchanger 3- air cooler 4- water cooler 5- alcohol 6- hydrogen reclaimer 7- circulator 8- steam drum separator 9- water pump 1a- water cooling 1b-gas cooling 11- cylinder body reactor reactor 12- heat-exchange 13- winding tube 14- inlet tube case 15- outlet tube case winding tube mandrel 16- upper seal 17-lower seal head 18-gas inlet 19-gas outlet head 20- positioning 21-top steam drum 22a- man hole on 22b- man hole on strip steam drum upper seal head 22b- man hole on 23- tube sheet 24- steam outlet 25- porous gas lower seal head collecting cylinder 26- central gas 27- porous gas 28- central gas 29- intermediate distributing distributing cylinder collecting cylinder sleeve cylinder 30- supporting rod 31- mixer 32- absorption 33- $CO_2$ removal Tower tower L1-raw gas L2- output gas from L3- released gas L4- circulating gas tower L5- fuel gas 34- gas distributing passage

## Detailed Description

The invention will be illustrated in detail with reference to the drawings and the following examples.

Example 1

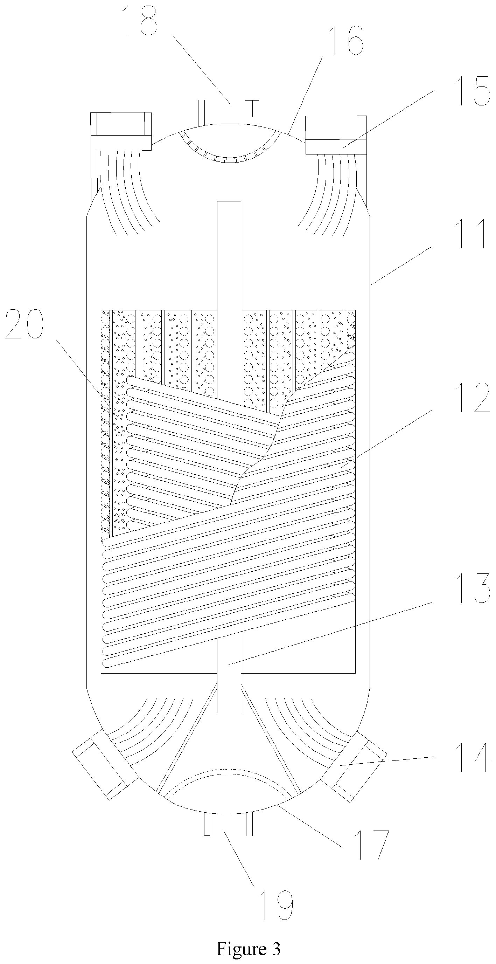

Fig. 1 shows a large loop for synthesis of methanol, consisting of a single vertical synthesis tower with a water cooling winding tube, and devices for heat exchange, separation and circulation, etc. The reactor 1 is a vertical reactor with a water cooling winding tube, the structure of which is shown in Fig. 3. The inlet line of the reactor 1 is connected to the shell side of the heat exchanger 2; the heat exchanger 2 is connected in the front to a circulator 7; the outlet line of the reactor 1 is connected to the tube side of the heat exchanger 2; the tube side outlet of the heat exchanger 2 is connected to an air cooler 3, a water cooler 4 and an alcohol separator 5; the outlet line of the alcohol separator 5 is divided into two lines, one connected to the circulator 7, and the other connected to a hydrogen reclaimer 6. The hydrogen reclaimer 6 is also connected to the circulator through a line. The reactor 1 is externally connected to a steam drum 8 and a water pump 9. After mixing, a raw gas L1 and a circulating gas L4 are heated to above 210 °C by an output gas L2 from the tower in the heat exchanger 2, and then enter the reactor 1 for synthesis of methanol. The reaction heat from the synthesis of methanol heats up water in the winding tube to produce medium-pressure steam. The output gas L2 from the tower is cooled to about 40 °C after passing through the heat exchanger 2, the air cooler 3 and the water cooler 4, and then enters the alcohol separator 5 for isolation of methanol. A part of the reaction gas with methanol isolated is returned as a circulating gas L4 through the circulator 7; another part is delivered as a released gas L3 to the hydrogen reclaimer 6 for reclaiming hydrogen which is fed back for synthesis of methanol; and the rest part L5 is output as a fuel gas, etc.

The reactor 1 used in Fig. 1 has a structure as shown in Fig. 3, comprising a housing, an internal heat-exchange member, an inlet tube case 14 and an outlet tube case 15, wherein the number of each of the inlet tube case 14 and the outlet tube case 15 is 2, and they are provided on a lower seal head 17 and an upper seal head 16 respectively. The internal heat-exchange member comprises a heat-exchange winding tube 12 which winds spirally around a mandrel 13 for the winding tube, and two ends of the heat-exchange winding tube are connected to the inlet tube case 14 and the outlet tube case 15 respectively, so as to form two heat-exchange tube bundles having different circular diameters with the tube winding mandrel as a circle center. The housing comprises the upper seal head 16, a cylinder body 11 and the lower seal head 17. A gas inlet 18 and a gas outlet 19 are provided on the upper seal head 16 and the lower seal head 17 respectively. The heat-exchange winding tube 12 is separated and positioned by a positioning strip 20 from inside to outside to form layer-by-layer tube sets, wherein a catalyst is loaded between the heat-exchange winding tubes to form a catalyst layer. Man holes are also provided on the upper seal head 16 and the lower seal head 17 for handling catalyst and maintenance. After heated by the heater 2, the mixture of the raw gas L1 and the circulating gas L2 shown in Fig. 1 enters the reactor 1 from the gas inlet 18 shown in Fig. 3, and particularly enters the catalyst layer to undergo reaction for synthesis of methanol. The reaction heat is absorbed by the water in the heat-exchange winding tube 12 to produce medium-pressure steam. After reaction, the gas exits the tower from the gas outlet 19. The inlet tube case 14 and the outlet tube case 15 of the reactor 1 are connected to the water pump 9 and the steam drum 8 respectively to form a loop.

Example 2

The large-scale loop for synthesis of methanol shown in Fig. 2 comprises a water cooling reactor 1a having a water cooling tube wound structure and a gas cooling reactor 1b having a gas cooling tube wound structure. The structure of the water cooling reactor 1a is the same as the reactor 1 described in Example 1. The gas cooling reactor 1b can be a reactor having the structure of the invention (e.g. reactor 1), or a gas cooling reactor having another type of structure, for example, as described in Chinese patent for invention ZL01808570.9 granted to the applicant of the present application.

The inlet tube case and the outlet tube case of the water cooling reactor 1a are connected to a water pump 9 and a steam drum 8 to form a loop. The shell-side gas inlet of the water cooling reactor 1a is connected to the tube-side outlet of the gas cooling reactor 1b. The shell-side gas outlet of the water cooling reactor 1a is connected to the shell-side inlet of the gas cooling reactor 1b. The shell-side outlet of the gas cooling reactor 1b is connected to a heat exchanger 2, a water cooler 4 and an alcohol separator 5 sequentially. The outlet line of the alcohol separator 5 is divided into two lines, one connected to a circulator 7, and the other connected to a hydrogen reclaimer 6. The hydrogen recycler 6 is also connected to the circulator through a line. The tube-side inlet of the gas cooling reactor 1b is connected to the circulator 7.

The water cooling reactor 1a and the gas cooling reactor 1b both use a tube wound structure. They are different in that the heat exchange medium in the heat-exchange winding tube of the water cooling reactor 1a is water, and the heat-exchange winding tube is connected to a steam drum and a water pump via the inlet tube case and the outlet tube case respectively to produce steam, while the heat exchange medium in the heat-exchange winding tube of the gas cooling reactor 1b is the raw gas, and the tube-side inlet and the tube-side outlet of the gas cooling reactor 1b are the inlet and outlet for the heat exchange medium. The shell-side inlet and the shell-side outlet are the inlet and outlet for the reaction gas.

After mixing, the raw gas L1 and the circulating gas L4 first enter the gas cooling reactor 1b from the tube-side inlet and are heated by an output gas L2 from the tower in the heat-exchange winding tube of the gas cooling reactor 1b. Then, the mixture exits the gas cooling reactor 1b from the tube-side outlet, enters the water cooling reactor 1a from the shell-side, and undergoes reaction for synthesis of methanol in the catalyst layer in the water cooling reactor 1a. The reaction heat from the synthesis of methanol heats up water in the heat-exchange winding tube to produce medium-pressure steam. The output gas L2 from the tower enters the gas cooling reactor 1b from the shell-side inlet, reacts in the catalyst layer outside the tube in the gas cooling reactor 1b for further synthesis of methanol, heats the raw gas in the heat-exchange winding tube, exists the tower from the shell-side outlet, passes through the heat exchanger 2, the water cooler 4 to be cooled to about 40 °C, and then enters the alcohol separator 5 for isolation of methanol. A part of the reaction gas with methanol isolated is returned as a circulating gas L4 through the circulator 7; another part is delivered as a released gas L3 to the hydrogen reclaimer 6 for reclaiming hydrogen which is fed back for synthesis of methanol.

After hydrogen is reclaimed, the rest gas is output as a fuel gas L5.

Example 3

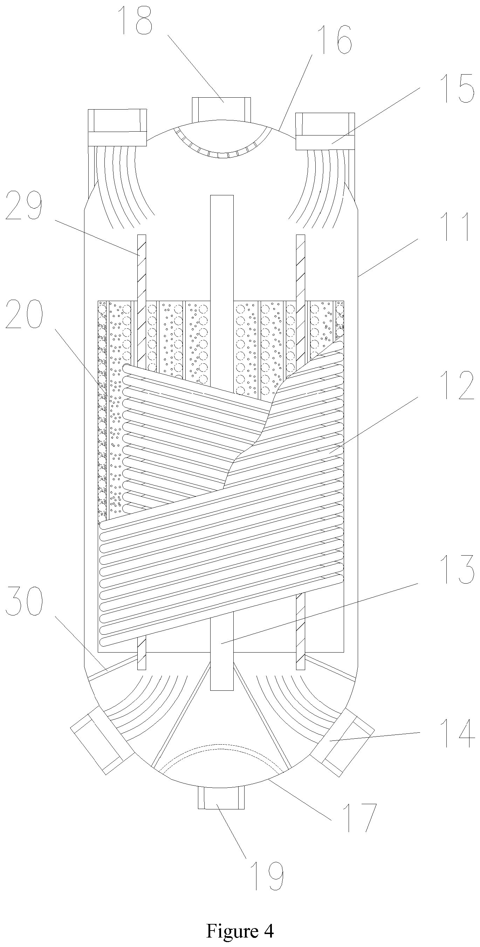

The large reactor shown in Fig. 4 is mostly the same as that shown in Fig. 3 in terms of structure, except that an intermediate sleeve 29 is provided to separate the heat-exchange winding tube sets to form an inner catalyst layer and an outer catalyst layer. A plurality of supporting rods 30 are provided at one end of the intermediate sleeve 29 to connect the seal head. The intermediate sleeve has a diameter which is half of the diameter of the housing.

Example 4

The axial water cooling tube wound reactor shown in Fig. 5 comprises a housing, an internal heat-exchange member and an inlet tube case 14, wherein there are two inlet tube cases 14 positioned symmetrically on the lower seal head 17. An upper seal head 16, a cylinder body 11 and the lower seal head 17 are provided on the housing. Atop steam drum 21, which is also used as an outlet tube case, is provided on the top of the upper seal head 16. Two winding tube sets spaced by about 500mm in a sectorial tube distribution zone are connected to a tube sheet 23 between the top steam drum 21 and the upper seal head 16. A gas inlet 18 and an upper-seal-head man hole 22b are provided at the side of the upper seal head 16. A steam outlet 24 and a steam-drum man hole 22a are provided on the top steam drum 21. The steam-drum man hole 22a is used for maintenance, and the upper-seal-head man hole 22b is used for loading catalyst and maintenance. A gas outlet 19 and a lower-seal-head man hole (not shown) for unloading catalyst and maintenance are provided on the lower seal head 17. The internal heat-exchange member comprises a heat-exchange winding tube 12 which winds spirally around a mandrel 13 for the winding tube, and two ends of the heat-exchange winding tube are connected to the inlet tube case 14 and a sectorial tube set in the tube sheet 23 respectively, so as to form heat-exchange tube bundles having different circular diameters with the tube winding mandrel as a circle center. The heat-exchange winding tube 12 is separated and positioned by a positioning strip 20 from inside to outside to form layer-by-layer tube sets, wherein a catalyst is loaded between the heat-exchange winding tubes to form a catalyst layer.

Using the device of Fig. 1, after mixing, the raw gas L1 and the circulating gas L2 enter the reactor 1 from the gas inlet 18 shown in Fig. 5, and particularly enter the catalyst layer to undergo reaction for synthesis of methanol. After reaction, the gas exits the tower from the gas outlet 19. The heat-exchange medium, water, enters the heat-exchange winding tube 12 from the inlet tube case 14, and moves upward spirally along the heat-exchange winding tube 12. The reaction heat from the synthesis of methanol is absorbed by the water in the heat-exchange winding tube 12 to produce medium-pressure steam which enters the top steam drum 21 and exits the reactor from the steam outlet 24.

Example 5

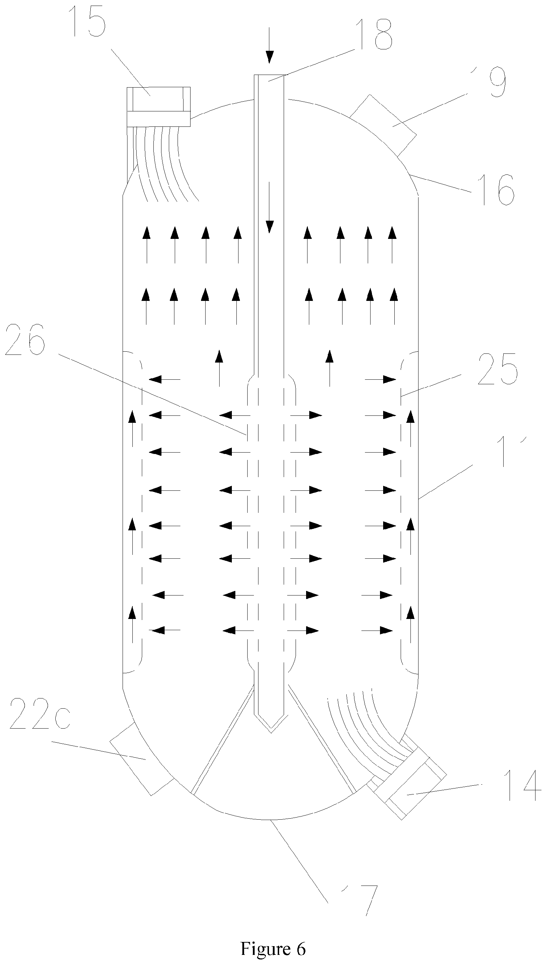

The axial-radial tube wound reactor with radial winding followed by axial winding as shown in Fig. 6 comprises a housing, an internal heat-exchange member, an inlet tube case 14 and an outlet tube case 15, wherein the number of each of the inlet tube case 14 and the outlet tube case 15 is 2, and they are provided on a lower seal head 17 and an upper seal head 16 respectively. The internal heat-exchange member comprises a heat-exchange winding tube which winds spirally, and two ends of the heat-exchange winding tube are connected to the inlet tube case 14 and the outlet tube case 15 respectively, so as to form heat-exchange tube bundles having different circular diameters with a tube winding mandrel as a circle center. The housing comprises the upper seal head 16, a cylinder body 11 and the lower seal head 17. A gas outlet 19 are provided on the upper seal head 16 (on the upper seal head, there is also provided an upper-seal-head man hole for loading catalyst and maintenance, not shown in the figure due to the limited room). The heat-exchange winding tube 12 is separated and positioned by a positioning strip from inside to outside to form layer-by-layer tube sets (the heat-exchange winding tube in the cylinder body is not shown in Fig. 6 for the purpose of showing clearly the flowing directions of the gas in this example), wherein a catalyst is loaded between the heat-exchange winding tubes to form a catalyst layer. A lower-seal-head man hole 22c is provided on the lower seal head 17 for unloading catalyst and maintenance.

A porous gas collecting cylinder 25 is provided at a middle-to-lower part of an inner wall of the cylinder body 11, and a central gas distributing cylinder 26 is provided at a center of the cylinder body, wherein the central gas distributing cylinder 26 is a hollow structure. The central gas distributing cylinder 26 is a hollow mandrel with its bottom sealed, and its top is a gas inlet 18. The central gas distributing cylinder 26 has pores at a middle-to-lower part, wherein a stainless steel screen (not shown) is laid on the central gas distributing cylinder 26 and the porous gas collecting cylinder 25 at a side adjacent to the catalyst, wherein the catalyst layer external to the heat-exchange winding tube is between the central gas distributing cylinder 26 and the porous gas collecting cylinder 25. (In order to show the gas outlet 19 and the lower-seal-head man hole 22c, Fig. 6 only shows one inlet tube case 14 and one outlet tube case 15; they each are actually present in a pair.)

A gas enters from the gas inlet 18 at a top of the central gas distributing cylinder 26 and is distributed uniformly through the distributing pores at the middle-to-lower part of the central gas distributing cylinder 26 into the catalyst layer for reaction, wherein most of the gas passes radially through the catalyst layer, enters the porous gas collecting cylinder 25, and flows upward in the porous gas collecting cylinder 25, wherein the reaction gas exiting the porous gas collecting cylinder 25 and a small portion of the gas exiting the central gas distributing cylinder 26 continue flowing upward axially to leave the reactor from the gas outlet 19.

Example 6

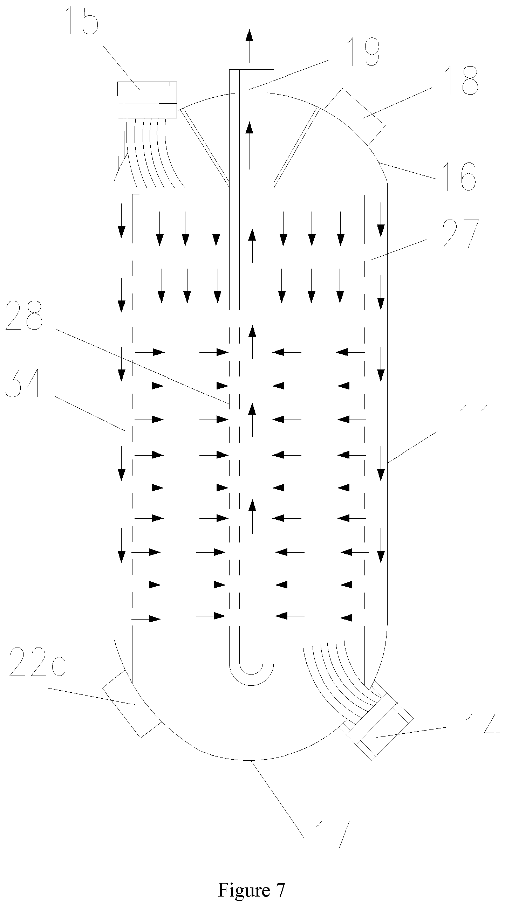

The axial-radial tube wound reactor with axial winding followed by radial winding shown in Fig. 7 comprises a housing, an internal heat-exchange member, an inlet tube case 14 and an outlet tube case 15, wherein the housing comprises an upper seal head 16, a cylinder body 11 and a lower seal head 17, wherein the outlet tube case 15, a gas inlet 18 and an upper-seal-head man hole (not shown) for loading catalyst and maintenance are provided on the upper seal head 16, wherein the inlet tube case 14 and a lower-seal-head man hole 22c for unloading catalyst and maintenance are provided on the lower seal head 17. The number of the inlet tube case 14 is two, and the number of the outlet tube case 15 is 4, provided on the lower seal head 17 and the upper seal head 16 respectively (in order to show the gas inlet 18 and the lower-seal-head man hole 22c, Fig. 6 only shows one inlet tube case 14 and one outlet tube case 15; they each are actually present in one pair and two pairs respectively). The internal heat-exchange member comprises a heat-exchange winding tube which winds spirally, and two ends of the heat-exchange winding tube are connected to the inlet tube case 14 and the outlet tube case 15 respectively, so as to form heat-exchange tube bundles having different circular diameters with the tube winding mandrel as a circle center (the heat-exchange winding tube in the cylinder body is not shown in Fig. 6 for the purpose of showing clearly the flowing directions of the gas in this example). A catalyst is loaded between the heat-exchange winding tubes to form a catalyst layer.

A porous gas distributing cylinder 27 is provided in an inner wall of the cylinder body 11, wherein except for 5% of the length of the porous gas distributing cylinder starting from the top end, pores are made along the rest 95% of the length at the middle-to-lower part, wherein a central gas collecting cylinder 28 is provided at a center of the cylinder body, wherein the central gas collecting cylinder 28 has pores at a middle-to-lower part which accounts for 90% of the length of the central gas collecting cylinder 28 starting from the lower end, wherein a stainless steel screen (not shown) is laid on the central gas collecting cylinder 28 and the porous gas distributing cylinder 27 at a side adjacent to the catalyst layer, wherein the central gas collecting cylinder 28 is fixed at a center of the cylinder body with a positioning ring, wherein the catalyst layer external to the heat-exchange winding tube is between the central gas collecting cylinder 28 and the porous gas distributing cylinder 27, wherein the gas inlet 18 is positioned on the upper seal head, wherein the gas outlet 19 is positioned at the top of the central gas collecting cylinder 28.

The reactor in this configuration is an axial-radial tube wound reactor with tubes wound axially first and then radially, wherein a gas enters from the gas inlet 18 on the upper seal head 16, wherein most of the gas flows downward along a gas distributing passage 34 between the housing and the porous gas distributing cylinder 27, and is distributed uniformly through the distributing pores in the porous gas distributing cylinder 27 into the catalyst layer, flows radially through the catalyst layer for reaction, and enters the central gas collecting cylinder 28 from collecting pores in the central gas collecting cylinder 28, wherein a small portion of the gas close to the center flows directly downward axially along an annular space extending along 10% of a height of a bed layer from a top thereof between the porous gas distributing cylinder 27 and the central gas collecting cylinder 28, and flows inward radially to converge in the inner gas collecting cylinder, and then flows upward in the central gas collecting cylinder 28 to exit the reactor from the gas outlet 19 at the top.

Example 7

The axial reactor shown in Fig. 3 according to the invention was used for producing megamethanol at 5000 tons/day, wherein the synthesis pressure was 8.2MPa, the diameter of the reactor was 5.8 meters, and the height of the catalyst bed layer was 7.5 meters. A catalyst for synthesis of methanol available from Nanjing Shide Co. was used, wherein the loading of the catalyst was $172M^3$. The space velocity was 6270/hour, the circulating ratio was 1.28, the

$$\frac{H_2 - CO_2}{CO + CO_2} = 1.9,$$

flow rate of the raw gas was $46 \times 10^4 NM^3/h$, the flow rate of the gas entering the tower was $107.8 \times 10^4 NM^3/h$, and the superficial velocity was $6271NM^3/h$. The temperature of the mixed gas was raised to 210 °C or higher by heat exchange with the gas exiting the synthesis tower, and the mixed gas entered the methanol tower for synthesis of methanol at about 250 °C. The flow rate of the gas exiting the tower was $78.5 \times 10^4 NM^3/h$. The gas exiting the tower contained 19.1% methanol. The pressure drop of the reactor was 0.13MPa. The total CO conversion was 97.4%, and the total $CO_2$ conversion was 95.8%. Heat was recovered from the gas exiting the tower by heat exchange. The gas exiting the tower had a temperature of about 230 °C, was cooled to 40 °C with water, and entered the methanol separator to separate the liquid phase containing about 98% methanol and 2% water. The crude methanol product was sent to be rectified to provide fine methanol at 5024 tons/day. Most of the gaseous phase was used as a circulating gas which was pressurized and mixed with the raw gas for further synthesis of methanol, and the other part in the form of a released gas was used to reclaim hydrogen by membrane separation, wherein the hydrogen was fed back for synthesis of methanol, and the rest part was utilized as a fuel gas. The raw gas was consumed at $2265NM^3/ton$ alcohol. The reaction heat from the synthesis of methanol heated the boiler water in the winding tube of the reactor to produce 2-4 MPa medium-pressure steam at 1.3 ton steam/ton alcohol.

Example 8

The axial-radial reactor shown in Fig. 7 according to the invention was used for producing megamethanol at 10000 tons/day, wherein the synthesis pressure was 8.2MPa, the diameter of the reactor was 5.4 meters, and the height of the catalyst bed layer was 18.8 meters. 356M$^3$ of a domestic catalyst (Nanjing N306 or Chengdu XN98) was used. The space velocity was 6742/hour, the flow rate of the raw gas was 97×10$^4$NM$^3$/h, and the hydrogen to carbon ratio of

the raw gas $\dfrac{H_2 - CO_2}{CO + CO_2} = 2.02$.   The raw gas was mixed with a circulating gas at a circulating ratio of 1.47. The flow rate of the gas entering the tower was 240×10$^4$NM$^3$/h. The temperature of the mixed gas was raised to 220 °C by heat exchange with the gas exiting the synthesis tower, and the mixed gas entered the reactor of the invention for synthesis of methanol at about 250 °C. The flow rate of the gas exiting the tower from the bottom was 179.5×10$^4$NM$^3$/h. The gas exiting the tower contained 17.2% methanol. The total CO conversion was 99.3%, and the total $CO_2$ conversion was 94%. The temperature was about 230 °C. Heat was recovered from the gas exiting the tower by heat exchange. The gas exiting the tower was cooled to 40 °C with water, and entered the methanol separator to separate the liquid phase. The crude methanol product containing about 96.3% methanol was sent to be rectified to provide fine methanol at 10369 tons/day. Most of the gaseous phase was used as a circulating gas which was mixed with the raw gas for further synthesis of methanol, and a small part in the form of a released gas was used to reclaim hydrogen by membrane separation, wherein the hydrogen was fed back for synthesis of methanol, and the rest part was utilized as a fuel gas. The raw gas was consumed at 2265NM$^3$/ton alcohol. The reaction heat from the synthesis of methanol heated the boiler water in the winding tube of the reactor to produce 2-4 MPa medium-pressure steam at 1.5 ton steam/ton alcohol. The pressure drop of the reactor was <0.1MPa.

Example 9

A large-scale process for synthesis of ethylene oxide by ethylene oxidation using a large water cooling tube wound reactor having an inner diameter of 4.2 m according the process flow shown in Fig. 8 included the following steps:

1) adding a $CH_4$ stabilizer and pure oxygen to an ethylene raw material purified to remove explosive alkane components including $H_2$, alkenes of $C_3$ or above to obtain a mixed raw gas comprising mainly ethylene 29%, oxygen 8%, methane 62%, $CO_2$ 1%;
2) mixing the raw gas L1 and a circulating gas L4 in a mixer 31, wherein the resulting mixture was heated to about 180 °C in a heat exchanger 2 at a pressure level of 2 MPa, and then entered a large reactor 1 for synthesis of ethylene oxide;
3) allowing the gas entering the tower to undergo reaction of ethylene oxidation to produce ethylene oxide on 120M$^3$ S-882 gaseous phase silver catalyst (available from SHELL Co.) comprising 13.9wt% silver and the balance of a co-catalyst and a support between the heat-exchange tube and the housing of the reactor 1, wherein oxidation reaction heat was absorbed by water in the tube to produce a medium-pressure steam, wherein the temperature of the oxidation reaction was controlled by controlling the pressure of the steam, wherein the space-time yield of the catalyst was 185kg/M$^3$•h, wherein the annual yield of ethylene oxide was 0.18 million tons;
4) lowering the temperature of the reaction gas exiting the reactor 1 to 40 °C by cooling it as the reaction gas heated the gas entering the reactor 1 with heat transferred in a hot gas passage in a heat exchanger 2;
5) allowing the reaction gas exiting the heat exchanger 2 to enter an absorption tower 32 to absorb ethylene oxide as a product, wherein a $C_2H_4O$ rich liquid at the bottom of the absorption tower was delivered to a post treatment station, wherein a small amount of released gas was discharged intermittently from a gas at the top of the absorption tower to avoid inert gas accumulation, and a small part of the gas passed through a $CO_2$ removing tower 33 and was pressurized in a circulator 7 together with the rest of the gas without removal of $CO_2$, wherein the pressurized gas returned to merge with the raw gas L1 in front of the reactor and then entered the reactor for synthesis of ethylene oxide.

Example 10

A large methanol reaction device consisted of two 5000 tons/day large methanol synthesis units in series, wherein each large methanol synthesis unit comprised a large water-cooling reactor, a heat exchanger, an air or water cooler, a methanol separator and a circulator, wherein 97*10$^3$NM$^3$/h fresh raw gas was all fed to the first methanol synthesis unit for synthesis of methanol at a circulating ratio of 0.134, wherein, except for a portion of an export gas from the methanol separator that was recycled through the circulator to the large reactor for further synthesis of methanol, the rest released gas was discharged into the second methanol synthesis unit at the back in series at a circulating ratio of 2 for synthesis of methanol, wherein a released gas from the second methanol synthesis unit was delivered to an effective gas (hydrogen) reclaiming device to reclaim hydrogen, wherein the reclaimed hydrogen was merged with the fresh raw gas, and then pressurized and delivered to the first synthesis unit for synthesis of methanol. The following table shows the comparative data of this example and the Comparative Example comprising two systems in parallel.

|  | Comparative Example Two systems in parallel | Two systems in series | | |
| --- | --- | --- | --- | --- |
|  |  | Front system | Released gas from the front system supplemented the back system | Total |
| I. Fresh gas flow $10^4 NM^3/h$ | 97 | 97 | 52.2 | 97 |
| Inert gas content % | 0.5 | 0.5 | 0.93 |  |
| II. Pressure of gas entering the tower MPa | 8.2 | 8.2 | 8.1 |  |
| $10^4 NM^3/h$ | 240 | 110 | 156.6 |  |
| Inert gas content % | 7 | 075 | 6.7 |  |
| Space velocity $h^{-1}$ | 6993 | 8720 | 8374 |  |
| III. Circulating gas flow $10^4 NM^3/h$ | 143 | 13 | 104.4 | 117.4 |
| Circulating ratio | 1.47 | 0.134 | 2 |  |
| IV. Methanol content in the gas exiting the methanol tower % | 16.88 | 18.58 | 12.53 |  |
| V. Amount of catalyst $M^3$ | 343 | 126 | 187 | 313 |
| Methanol yield normalized to 100% ton/day | 10240 | 5032 | 5208 | 10240 |

As can be seen from the table, with the two systems in series of the invention used, the circulating gas flow and the inert gas content in the front system were reduced greatly, and a daily yield of 5000 tons of methanol was achieved with a smaller amount of catalyst. Only the circulating gas flow and the inert gas content in the back system were similar to the tower operating in a parallel mode. As a result, when the same amount of fresh gas was consumed, the serial mode produces the same amount of methanol as the parallel mode using a lower total circulating gas flow and less catalyst.

**Claims**

1. A large reactor, comprising a housing, an internal heat-exchange member, an inlet tube case (14) and an outlet tube case (15), wherein the internal heat-exchange member comprises a heat-exchange winding tube; wherein the housing comprises an upper seal head (16), a cylinder body, a lower seal head (17); wherein a gas inlet (18) and a gas outlet (19) are provided on the upper seal head (16) and the lower seal head (17) respectively; wherein the numbers of the inlet tube case (14) and/or the outlet tube case (15) each are 2 or a multiple of 2; wherein the inlet tube case (14) and the outlet tube case (15) are connected to two ends of the heat-exchange winding tube respectively, wherein the heat-exchange winding tube is separated and positioned by a positioning strip (20) from inside to outside and winds into a layer-by-layer tube set, wherein a catalyst is loaded between heat-exchange winding tubes to form a catalyst layer, **characterized in that** the inlet tube case (14) and/or the outlet tube case (15) are respectively provided on the lower seal head (17) and the upper seal head (16), or on a lower part and an upper part of the cylinder body, arranged uniformly in a circumferentially symmetric manner, and wherein a steam drum is provided on the upper seal head (16) and is also used as an outlet tube case (15), wherein an even number of winding tube sets in a sectorial tube distribution zone are connected to a tube sheet between the steam drum and the upper seal head (16).

2. The large reactor of claim 1, wherein the large reactor is a radial-axial reactor, wherein a porous gas collecting cylinder (25) is provided at a middle-to-lower part of an inner wall of the cylinder body, and a central gas distributing cylinder (26) is provided at a center of the cylinder body, wherein the central gas distributing cylinder (26) has pores at a middle-to-lower part, wherein a stainless steel screen is laid on the central gas distributing cylinder (26) and the porous gas collecting cylinder (25) at a side adjacent to the catalyst, wherein the catalyst layer external to the heat-exchange winding tube is between the central gas distributing cylinder (26) and the porous gas collecting cylinder (25), wherein the gas outlet (19) is positioned on the upper seal head (16), wherein the gas inlet (18) is positioned at a top of the central gas distributing cylinder (26), wherein the central gas distributing cylinder (26) is a hollow mandrel with its bottom sealed.

3. The large reactor of claim 1, wherein the large reactor is an axial-radial reactor, wherein a porous gas distributing cylinder (27) is provided in an inner wall of the cylinder body; wherein the porous gas distributing cylinder (27) has pores at a middle-to-lower part which accounts for 90-95% of a length of the porous gas distributing cylinder (27) starting from a lower end, wherein a central gas collecting cylinder (28) is provided at a center of the cylinder body, wherein the central gas collecting cylinder (28) has pores at a middle-to-lower part which accounts for 90-95% of a length of the central gas collecting cylinder (28) starting from a lower end, wherein a stainless steel screen is laid on the central gas collecting cylinder (28) and the porous gas distributing cylinder (27) at a side adjacent to the catalyst layer, wherein the central gas collecting cylinder (28) is fixed at a center of the cylinder body with a positioning ring, wherein the catalyst layer external to the heat-exchange winding tube is between the central gas collecting cylinder (28) and the porous gas distributing cylinder (27), wherein the gas inlet (18) is positioned on the upper seal head (16), wherein the gas outlet (19) is positioned at a top of the central gas collecting cylinder (28).

4. The large reactor of claim 1, wherein the heat-exchange winding tube is a small-size spiral heat-exchange tube having an inner diameter of 10-25 mm.

5. The large reactor of claim 1, wherein the heat-exchange winding tube set is separated by an intermediate sleeve (29) or a perforated sleeve to provide an inner catalyst layer and an outer catalyst layer, wherein an end of the intermediate sleeve (29) or perforated sleeve is connected to the seal head via a plurality of supporting rods (30), wherein the intermediate sleeve (29) or perforated sleeve has a diameter of 1/2 to 1/3 of the diameter of the housing.

6. A large reaction device comprising the large reactor of claim 1, wherein the large reaction device comprises a single large axial water cooling tube wound reactor, or two or three large axial water cooling tube wound reactors in parallel, or a large axial water cooling tube wound reactor connected to a large gas-cooling reactor in series.

7. A large-scale process for synthesis of methanol using the large reactor of claim 1, **characterized by** a low circulating ratio of <3 and a high net value of >10%, wherein the process includes the following steps specifically:

   1) mixing a fresh raw gas with hydrogen reclaimed from a released gas from the synthesis to form a mixed gas,

   $$\frac{(H_2 - CO_2)}{(CO + CO_2)} = 2.0\text{-}2.05,$$
   wherein wherein the mixed gas is purified deeply and mixed with a circulating gas;
   2) raising a temperature of the mixed gas to above 210°C by heat exchange in a heat exchanger and feeding it to the large reactor for synthesis of methanol, wherein methanol in the reaction gas at the outlet has a molar percentage of >10%, wherein reaction heat from the synthesis of methanol heats up water in the heat-exchange winding tube to produce a steam;
   3) cooling a hot gas having a high content of methanol exiting the large reactor by heat exchange in a heat exchanger, wherein the cooling is achieved by heating water in a boiler or with an air cooler, wherein the gas is then cooled to 40 °C by water cooling, and then delivered to an alcohol separator for isolation of methanol;
   4) recycling the reaction gas after product separation through a circulator to undergo further reaction, wherein the ratio of the circulating gas to the fresh raw gas, namely the circulating ratio, is less than 3, wherein, as another part, the released gas is subjected to membrane separation, pressure swing absorption or the like to reclaim effective hydrogen which returns to synthesize a product, or enters a next methanol synthesis reaction loop for further reaction to form methanol, or the released gas is used for co-production of other products or for power generation.

8. A methanol synthesis device comprising the large reactor of claim 1, wherein the device has two large reaction units in series, wherein each large reaction unit comprises a large reactor, a heat exchanger, an air or water cooler, a methanol separator and a circulator.

9. A large device for synthesis of ethylene oxide by ethylene oxidation comprising the large reactor of claim 1, wherein the large device for synthesis of ethylene oxide by ethylene oxidation comprises a reactor (1), a heat exchanger (2), a mixer (31), an absorption tower (32), a $CO_2$ removing tower (33), a circulator (7), a steam drum (8) and a water pump (9), wherein the mixer (31) is connected to the heat exchanger (2), the reactor (1), the absorption tower (32), the circulator (7) sequentially, wherein the outlet of the reactor (1) is connected to the tube-side or shell-side inlet of the heat exchanger (2), wherein the tube-side or shell-side outlet of the heat exchanger (2) is connected to the absorption tower (32), wherein the outlet of the absorption tower (32) is divided into two lines, one connected to the circulator (7) and the other connected to the $CO_2$ removing tower (33), wherein the reactor (1) is externally connected to the steam drum (8) and the water pump (9) to produce steam.

## Patentansprüche

1. Großer Reaktor, umfassend ein Gehäuse, ein inneres Wärmeaustauschelement, eine Einlassröhrenhülle (14) und eine Auslassröhrenhülle (15), wobei das innere Wärmeaustauschelement eine Wärmeaustauschwicklungsröhre umfasst; wobei das Gehäuse einen oberen Dichtungskopf (16), einen Zylinderkörper, einen unteren Dichtungskopf (17) umfasst; wobei ein Gaseinlass (18) und ein Gasauslass (19) jeweils an dem oberen Dichtungskopf (16) und dem unteren Dichtungskopf (17) vorgesehen sind; wobei die Anzahl der Einlassröhrenhülle (14) und/oder der Auslassröhrenhülle (15) jeweils 2 oder ein Vielfaches von 2 ist; wobei die Einlassröhrenhülle (14) und die Auslassröhrenhülle (15) jeweils mit zwei Enden der Wärmeaustauschwicklungsröhre verbunden sind, wobei die Wärmeaustauschwicklungsröhre durch einen Positionierungsstreifen (20) von innen nach außen getrennt und positioniert ist und sich zu einem schichtweisen Röhrensatz wickelt, wobei ein Katalysator zwischen Wärmeaustauschwicklungsröhren geladen ist, um eine Katalysatorschicht zu bilden, **dadurch gekennzeichnet, dass** die Einlassröhrenhülle (14) und/oder die Auslassrohrhülse (15) jeweils an dem unteren Dichtungskopf (17) und dem oberen Dichtungskopf (16) oder an einem unteren Teil und einem oberen Teil des Zylinderkörpers gleichmäßig umfangssymmetrisch angeordnet vorgesehen sind, und wobei eine Dampftrommel an dem oberen Dichtungskopf (16) vorgesehen ist und auch als Auslassröhrenhülle (15) verwendet wird, wobei eine gerade Anzahl von Wickelrohrsätzen in einer sektoriellen Rohrverteilungszone mit einem Rohrboden zwischen der Dampftrommel und dem oberen Dichtungskopf (16) verbunden ist.

2. Großer Reaktor nach Anspruch 1, wobei der große Reaktor ein Radial-Axial-Reaktor ist, wobei ein poröser Gassammelzylinder (25) an einem mittleren bis unteren Teil einer Innenwand des Zylinderkörpers vorgesehen ist, und ein zentraler Gasverteilungszylinder (26) in der Mitte des Zylinderkörpers vorgesehen ist, wobei der zentrale Gasverteilungszylinder (26) Poren an einem mittleren bis unteren Teil aufweist, wobei ein Edelstahlsieb auf den zentralen Gasverteilungszylinder (26) und den porösen Gassammelzylinder (25) an einer Seite neben dem Katalysator gelegt ist, wobei die Katalysatorschicht außerhalb der Wärmeaustauschwicklungsröhre zwischen dem zentralen Gasverteilungszylinder (26) und dem porösen Gassammelzylinder (25) liegt, wobei der Gasauslass (19) auf dem oberen Dichtungskopf (16) positioniert ist, wobei der Gaseinlass (18) an einem oberen Ende des zentralen Gasverteilungszylinders (26) positioniert ist, wobei der zentrale Gasverteilungszylinder (26) ein hohler Dorn ist, dessen Boden abgedichtet ist.

3. Großer Reaktor nach Anspruch 1, wobei der große Reaktor ein Axial-Radial-Reaktor ist, wobei ein poröser Gasverteilungszylinder (27) in einer Innenwand des Zylinderkörpers vorgesehen ist; wobei der poröse Gasverteilungszylinder (27) Poren an einem mittleren bis unteren Teil aufweist, der 90-95 % einer Länge des porösen Gasverteilungszylinders (27), beginnend von einem unteren Ende, ausmacht, wobei ein zentraler Gassammelzylinder (28) in einer Mitte des Zylinderkörpers vorgesehen ist, wobei der zentrale Gassammelzylinder (28) Poren an einem mittleren bis unteren Teil aufweist, der 90-95 % einer Länge des zentralen Gassammelzylinders (28), beginnend von einem unteren Ende, ausmacht, wobei ein Edelstahlsieb auf den zentralen Gassammelzylinder (28) und den porösen Gasverteilungszylinder (27) an einer Seite neben der Katalysatorschicht gelegt ist, wobei der zentrale Gassammelzylinder (28) in einer Mitte des Zylinderkörpers mit einem Positionierungsring befestigt ist, wobei sich die Katalysatorschicht außerhalb der Wärmeaustauschwicklungsröhre zwischen dem zentralen Gassammelzylinder (28) und dem porösen Gasverteilungszylinder (27) befindet, wobei der Gaseinlass (18) an dem oberen Dichtungskopf (16) positioniert ist, wobei der Gasauslass (19) an einem oberen Ende des zentralen Gassammelzylinders (28) positioniert ist.

4.  Großer Reaktor nach Anspruch 1, wobei die Wärmeaustauschwicklungsröhre eine kleine spiralförmige Wärmeaustauschröhre mit einem Innendurchmesser von 10-25 mm ist.

5.  Großer Reaktor nach Anspruch 1, wobei der Wärmeaustauschwicklungsröhresatz durch eine Zwischenhülse (29) oder eine perforierte Hülse getrennt ist, um eine innere Katalysatorschicht und eine äußere Katalysatorschicht bereitzustellen, wobei ein Ende der Zwischenhülse (29) oder der perforierten Hülse über mehrere Stützstäbe (30) mit dem Dichtungskopf verbunden ist, wobei die Zwischenhülse (29) oder die perforierte Hülse einen Durchmesser von 1/2 bis 1/3 des Durchmessers des Gehäuses aufweist.

6.  Große Reaktionsvorrichtung, umfassend den großen Reaktor nach Anspruch 1, wobei die große Reaktionsvorrichtung einen einzelnen großen axialen Reaktor mit gewickeltem Wasserkühlungsrohr oder zwei oder drei große axiale Reaktoren mit gewickeltem Wasserkühlungsrohr, die parallel geschaltet sind, oder einen großen axialen Reaktor mit gewickeltem Wasserkühlungsrohr, der mit einem großen Gaskühlungsreaktor in Reihe verbunden ist, umfasst.

7.  Großtechnisches Verfahren zur Synthese von Methanol unter Verwendung des großen Reaktors nach Anspruch 1, **gekennzeichnet durch** ein niedriges Zirkulationsverhältnis von <3 und einen hohen Nettowert von >10 %, wobei das Verfahren insbesondere die folgenden Schritte einschließt:

    1) Mischen eines frischen Rohgases mit Wasserstoff, der aus einem aus der Synthese freigesetzten Gas

    $$\frac{(H_2 - CO_2)}{(CO + CO_2)} = 2.0\text{-}2.05$$

    zurückgewonnen wurde, um ein Mischgas zu bilden, wobei ' wobei das Mischgas tief gereinigt und mit einem zirkulierenden Gas gemischt wird;

    2) Erhöhen einer Temperatur des Mischgases auf über 210 °C durch Wärmeaustausch in einem Wärmetauscher und Zuführen des Gases in den großen Reaktor zur Synthese von Methanol, wobei Methanol im Reaktionsgas am Auslass einen Molprozentsatz von >10 % aufweist, wobei die Reaktionswärme aus der Synthese von Methanol Wasser im Wärmeaustauschwicklungsröhre erhitzt, um einen Dampf zu erzeugen;

    3) Kühlen eines heißen Gases mit einem hohen Gehalt an Methanol, das aus dem großen Reaktor austritt, durch Wärmeaustausch in einem Wärmetauscher, wobei die Kühlung durch Erhitzen von Wasser in einem Kessel oder mit einem Luftkühler erreicht wird, wobei das Gas dann durch Wasserkühlung auf 40 °C abgekühlt und dann zur Isolierung des Methanols einem Alkoholabscheider zugeführt wird;

    4) Rezyklieren des Reaktionsgases nach der Produkttrennung durch einen Zirkulator, um eine weitere Reaktion durchzuführen, wobei das Verhältnis des zirkulierenden Gases zum frischen Rohgas, d. h. das Zirkulationsverhältnis, weniger als 3 beträgt, wobei als weiterer Teil das freigesetzte Gas einer Membrantrennung, Druckwechselabsorption oder dergleichen unterzogen wird, um wirksamen Wasserstoff zurückzugewinnen, der zur Synthese eines Produkts zurückkehrt, oder in einen nächsten Methanolsynthese-Reaktionskreislauf zur weiteren Reaktion zur Bildung von Methanol eintritt, oder das freigesetzte Gas zur Koproduktion anderer Produkte oder zur Stromerzeugung verwendet wird.

8.  Methanolsynthesevorrichtung, umfassend den großen Reaktor nach Anspruch 1, wobei die Vorrichtung zwei große Reaktionseinheiten in Reihe aufweist, wobei jede große Reaktionseinheit einen großen Reaktor, einen Wärmetauscher, einen Luft- oder Wasserkühler, einen Methanolabscheider und einen Zirkulator umfasst.

9.  Große Vorrichtung zur Synthese von Ethylenoxid durch Ethylenoxidation, umfassend den großen Reaktor nach Anspruch 1, wobei die große Vorrichtung zur Synthese von Ethylenoxid durch Ethylenoxidation einen Reaktor (1), einen Wärmetauscher (2), einen Mischer (31), einen Absorptionsturm (32), einen $CO_2$-Entfernungsturm (33), einen Zirkulator (7), eine Dampftrommel (8) und eine Wasserpumpe (9) umfasst, wobei der Mischer (31) sequentiell mit dem Wärmetauscher (2), dem Reaktor (1), dem Absorptionsturm (32), dem Zirkulator (7) verbunden ist, wobei der Auslass des Reaktors (1) sequentiell mit dem rohrseitigen oder mantelseitigen Einlass des Wärmetauschers (2) verbunden ist, wobei der rohrseitige oder mantelseitige Auslass des Wärmetauschers (2) mit dem Absorptionsturm (32) verbunden ist, wobei der Auslass des Absorptionsturms (32) in zwei Leitungen geteilt ist, von denen eine mit dem Zirkulator (7) und die andere mit dem $CO_2$-Entfernungsturm (33) verbunden ist, wobei der Reaktor (1) extern mit der Dampftrommel (8) und der Wasserpumpe (9) verbunden ist, um Dampf zu erzeugen.

**Revendications**

1. Réacteur de grande taille, comprenant un logement, un élément d'échange de chaleur interne, un carter de tube d'entrée (14) et un carter de tube de sortie (15), dans lequel l'élément d'échange de chaleur interne comprend un tube d'enroulement d'échange de chaleur ; dans lequel le logement comprend une tête d'étanchéité supérieure (16), un corps cylindrique, une tête d'étanchéité inférieure (17); dans lequel une entrée de gaz (18) et une sortie de gaz (19) sont respectivement prévues sur la tête d'étanchéité supérieure (16) et la tête d'étanchéité inférieure (17) ; dans lequel les nombres du carter de tube d'entrée (14) et/ou du carter de tube de sortie (15) sont chacun 2 ou un multiple de 2 ; dans lequel le carter de tube d'entrée (14) et le carter de tube de sortie (15) sont connectés respectivement aux deux extrémités du tube d'enroulement d'échange de chaleur, dans lequel le tube d'enroulement d'échange de chaleur est séparé et positionné par une bande de positionnement (20) de l'intérieur vers l'extérieur et s'enroule dans un ensemble de tubes couche par couche, dans lequel un catalyseur est chargé entre des tubes d'enroulement d'échange de chaleur pour former une couche de catalyseur, **caractérisé en ce que** le carter de tube d'entrée (14) et/ou le carter de tube de sortie (15) sont respectivement prévus sur la tête d'étanchéité inférieure (17) et la tête d'étanchéité supérieure (16), ou sur une partie inférieure et une partie supérieure du corps cylindrique, agencé(s) uniformément d'une manière symétrique circonférentiellement, et dans lequel un tambour à vapeur est prévu sur la tête d'étanchéité supérieure (16) et est également utilisé comme carter de tube de sortie (15), dans lequel un nombre pair d'ensembles de tubes d'enroulement dans une zone de distribution de tubes sectorielle sont connectés à une feuille de tubes entre le tambour à vapeur et la tête d'étanchéité supérieure (16).

2. Réacteur de grande taille selon la revendication 1, dans lequel le réacteur de grande taille est un réacteur radial-axial, dans lequel un cylindre de collecte de gaz poreux (25) est prévu dans une partie médiane à inférieure d'une paroi intérieure du corps cylindrique, et un cylindre de distribution de gaz central (26) est prévu au centre du corps cylindrique, dans lequel le cylindre de distribution de gaz central (26) a des pores dans une partie médiane à inférieure, dans lequel un écran en acier inoxydable est placé sur le cylindre de distribution de gaz central (26) et le cylindre de collecte de gaz poreux (25) au niveau d'un côté adjacent au catalyseur, dans lequel la couche de catalyseur externe au tube d'enroulement d'échange de chaleur se trouve entre le cylindre de distribution de gaz central (26) et le cylindre de collecte de gaz poreux (25), dans lequel la sortie de gaz (19) est positionnée sur la tête d'étanchéité supérieure (16), dans lequel l'entrée de gaz (18) est positionnée à un sommet du cylindre de distribution de gaz central (26), dans lequel le cylindre de distribution de gaz central (26) est un mandrin creux dont le fond est scellé.

3. Réacteur de grande taille selon la revendication 1, dans lequel le réacteur de grande taille est un réacteur axial-radial, dans lequel un cylindre de distribution de gaz poreux (27) est prévu dans une paroi intérieure du corps cylindrique ; dans lequel le cylindre de distribution de gaz poreux (27) a des pores dans une partie médiane à inférieure qui représentent 90 à 95 % d'une longueur du cylindre de distribution de gaz poreux (27) en partant depuis une extrémité inférieure, dans lequel un cylindre de collecte de gaz central (28) est prévu au centre du corps cylindrique, dans lequel le cylindre de collecte de gaz central (28) a des pores au niveau d'une partie médiane à inférieure qui représentent 90 à 95 % d'une longueur du cylindre de collecte de gaz central (28) en partant d'une extrémité inférieure, dans lequel un écran en acier inoxydable est placé sur le cylindre de collecte de gaz central (28) et le cylindre de distribution de gaz poreux (27) au niveau d'un côté adjacent à la couche de catalyseur, dans lequel le cylindre de collecte de gaz central (28) est fixé au niveau du centre du corps cylindrique avec une bague de positionnement, dans lequel la couche de catalyseur externe au tube d'enroulement d'échange de chaleur se situe entre le cylindre de collecte de gaz central (28) et le cylindre de distribution de gaz poreux (27), dans lequel l'entrée de gaz (18) est positionnée sur la tête d'étanchéité supérieure (16), dans lequel la sortie de gaz (19) est positionnée au niveau d'un sommet du cylindre de collecte de gaz central (28).

4. Réacteur de grande taille selon la revendication 1, dans lequel le tube d'enroulement d'échange de chaleur est un tube d'échange de chaleur en spirale de petite taille ayant un diamètre intérieur de 10 à 25 mm.

5. Réacteur de grande taille selon la revendication 1, dans lequel l'ensemble de tubes d'enroulement d'échange de chaleur est séparé par un manchon intermédiaire (29) ou un manchon perforé pour fournir une couche de catalyseur interne et une couche de catalyseur externe, dans lequel une extrémité du manchon intermédiaire (29) ou du manchon perforé est connectée à la tête d'étanchéité via une pluralité de tiges de support (30), dans lequel le manchon intermédiaire (29) ou le manchon perforé a un diamètre de 1/2 à 1/3 du diamètre du logement.

6. Dispositif de réaction de grande taille comprenant le réacteur de grande taille selon la revendication 1, dans lequel le dispositif de réaction comprend un réacteur de grande taille axial d'enroulement de tube de refroidissement à eau unique, ou deux ou trois réacteurs axiaux d'enroulement de tube de refroidissement à eau en parallèle, ou un réacteur de grande taille axial d'enroulement de tube de refroidissement à eau connecté à un réacteur de grande taille de refroidissement par gaz en série.

7. Procédé à grande échelle de synthèse de méthanol utilisant le réacteur de grande taille de la revendication 1, **caractérisé par** un faible taux de circulation < 3 et une valeur nette élevée > 10 %, dans lequel le procédé comprend spécifiquement les étapes suivantes consistant à :

> 1) mélanger un gaz brut frais avec de l'hydrogène récupéré à partir d'un gaz libéré de la synthèse pour former un gaz, dans laquelle $\frac{(H_2-CO_2)}{(CO+CO_2)} = 2{,}0 \text{ à } 2{,}05,$ dans lequel le gaz mixte est purifié en profondeur et mélangé avec un gaz circulant ;
> 2) élever une température du gaz mixte jusqu'au-dessus de 210°C par échange de chaleur dans un échangeur de chaleur et l'alimenter vers le réacteur de grande taille pour une synthèse de méthanol, dans lequel du méthanol dans le gaz de réaction au niveau de la sortie a un pourcentage molaire de > 10%, dans lequel de la chaleur de réaction provenant de la synthèse de méthanol chauffe l'eau dans le tube d'enroulement d'échange de chaleur pour produire une vapeur ;
> 3) refroidir un gaz chaud à haute teneur en méthanol sortant du réacteur de grande taille par échange de chaleur dans un échangeur de chaleur, dans lequel le refroidissement est réalisé en chauffant de l'eau dans une chaudière ou avec un refroidisseur à air, dans lequel le gaz est ensuite refroidi à 40°C par refroidissement à l'eau, puis acheminé vers un séparateur d'alcool pour un isolement de méthanol ;
> 4) recycler le gaz de réaction après la séparation du produit à travers un dispositif de circulation pour subir une réaction supplémentaire, dans lequel le rapport du gaz en circulation au gaz brut frais, à savoir le rapport de circulation, est inférieur à 3, dans lequel, comme autre partie, le gaz libéré est soumis à une séparation membranaire, à une absorption modulée en pression ou similaire pour récupérer de l'hydrogène efficace qui retourne synthétiser un produit, ou entre dans une prochaine boucle de réaction de synthèse de méthanol pour une réaction supplémentaire pour former du méthanol, ou le gaz libéré est utilisé pour une co-production d'autres produits ou pour une production d'électricité.

8. Dispositif de synthèse de méthanol comprenant le réacteur de grande taille selon la revendication 1, dans lequel le dispositif a deux unités de réaction en série de grande taille, dans lequel chaque unité de réaction de grande taille comprend un réacteur de grande taille, un échangeur de chaleur, un refroidisseur à air ou eau, un séparateur de méthanol et un dispositif de circulation.

9. Dispositif de grande taille de synthèse d'oxyde d'éthylène par oxydation d'éthylène comprenant le réacteur de grande taille selon la revendication 1, dans lequel le dispositif de grande taille de synthèse d'oxyde d'éthylène par oxydation d'éthylène comprend un réacteur (1), un échangeur de chaleur (2), un mélangeur (31), une tour d'absorption (32), une tour d'élimination de $CO_2$ (33), un dispositif de circulation (7), un tambour à vapeur (8) et une pompe à eau (9), dans lequel le mélangeur (31) est connecté à l'échangeur de chaleur (2), au réacteur (1), à la tour d'absorption (32) et au circulateur (7) séquentiellement, dans lequel la sortie du réacteur (1) est connectée à l'entrée côté tube ou côté enveloppe de l'échangeur de chaleur (2), dans lequel la sortie côté tube ou côté enveloppe de l'échangeur de chaleur (2) est connectée à la tour d'absorption (32), dans lequel la sortie de la tour d'absorption (32) est divisée en deux lignes, l'une connectée au dispositif de circulation (7) et l'autre connecté à la tour d'élimination de $CO_2$ (33), dans lequel le réacteur (1) est connecté de manière externe au tambour à vapeur (8) et à la pompe à eau (9) pour produire de la vapeur.

Figure 1

Figure 2

Figure 3

Figure 4

Figure 5

Figure 6

Figure 7

Figure 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007021393 A
- CN 101243027
- CN 202460592 U
- CN 103240036
- CN 203096014 U
- CN 101301597 A
- WO 2004065341 A
- CN ZL01808570